# FASCICULE DE BREVET EUROPEEN

(11) **EP 2 124 823 B1**
(45) Date de publication et mention de la délivrance du brevet: **23.03.2011**
(21) Numéro de dépôt: 08775608.6
(22) Date de dépôt: 29.02.2008
(51) Int. Cl.: A61F 2/20

(54) **IMPLANT UNIVERSEL POUR THYROPLASTIE**
UNIVERSELLES IMPLANTAT FÜR THYROPLASTIK
UNIVERSAL IMPLANT FOR THYROPLASTY

(30) Priorité: 01.03.2007 FR 0701482
(43) Date de publication de la demande: 02.12.2009
(73) Titulaire: PROTIP SAS, 67380 Lingolsheim (FR); Université Louis Pasteur (ULP), 67000 Strasbourg Cedex (FR); Hôpitaux Universitaires de Strasbourg (HUS), 67000 Strasbourg (FR)
(72) Inventeur: WALDER, André, F-94240 L'Hay-Les-Roses (FR); DEBRY, Christian, F-75015 Paris (FR); SCHULTZ, Phillippe, F-67203 Oberschaeffolsheim (FR)
(74) Mandataire: Barny, Luc
(86) Numéro de dépôt international: PCT/FR2008/000262
(87) Numéro de publication internationale: WO 2008/129164

(56) Documents cités:
- EP-A- 0 453 186
- US-A- 5 197 982
- US-A- 5 306 298
- US-A- 5 693 096

## Description

La présente invention concerne le domaine des implants chirurgicaux et, plus particulièrement, le domaine des implants destinés à corriger une paralysie totale ou partielle des cordes vocales de manière à restituer la parole à un patient. De manière préférée, l'invention vise un implant universel et réglable utilisé en thyroplastie.

Parmi les paralysies vocales, peuvent être citées les paralysies unilatérales ayant principalement des conséquences phonatoires et les paralysies bilatérales entraînant, quant à elles, des problèmes respiratoires. Dans les deux cas, surviennent des complications au niveau de la déglutition.

La présente invention vise préférentiellement, mais sans y être limitée, les paralysies unilatérales.

Dans la pratique, de telles paralysies sont généralement dues à des traumatismes laryngés, des affections cancéreuses ou, plus rares, des atteintes infectieuses.

Les conséquences principales sont la dysphonie, c'est-à-dire une voix soufflée, peu ou pas sonorisée, due à une compensation uniquement partielle par la corde vocale non altérée. Il est alors nécessaire de pallier ces dysfonctionnements en limitant la fuite glottique et surtout en augmentant la tension de la corde vocale endommagée.

Il existe, pour ce faire, diverses méthodes visant à pallier un tel dysfonctionnement des cordes vocales. Peuvent être citées, par exemple, des techniques endoscopiques comme les injections de téflon, de collagène, de macroplastique ou encore de graisse. Si de telles injections sont rapides à mettre en oeuvre et reproductibles à volonté, elles ne donnent pas entière satisfaction car s'ensuit généralement une détérioration vocale dans le temps. En outre, les difficultés liées à l'injection entraînent très souvent une mauvaise répartition du produit qui est à l'origine d'une agglutination au point d'injection.

Plus récemment, ont été développées plusieurs techniques chirurgicales qui ont toutes comme principe commun l'insertion d'un implant via une incision, aussi appelée fenêtre, réalisée au sein du cartilage thyroïdien. Il peut être mentionné, à titre d'exemples de tels implants, les implants décrits pas Isshiki Nobuhiko et, plus récemment, l'implant décrit par Montgomery.

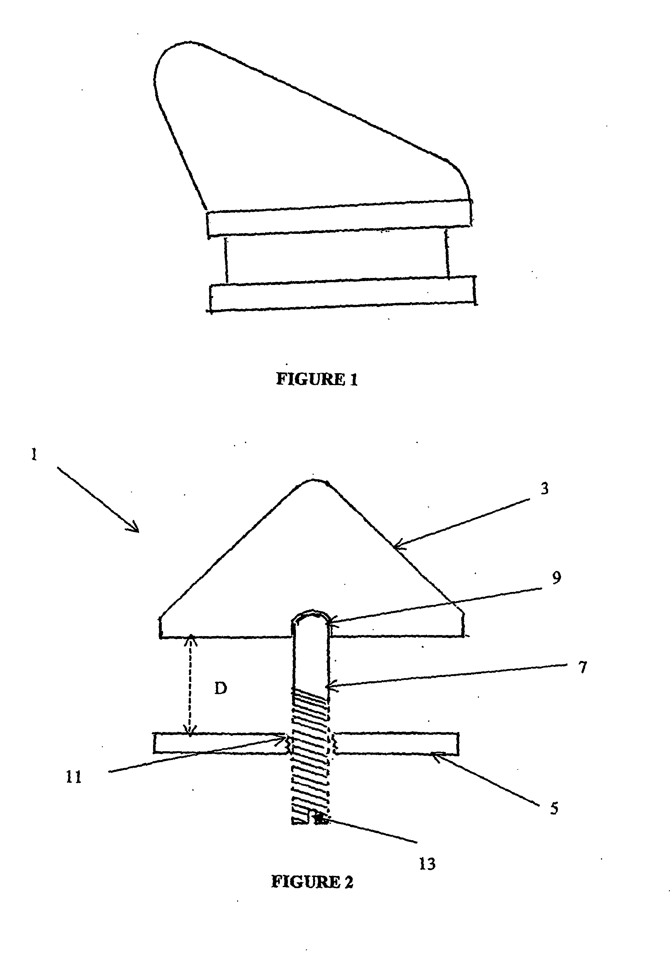

En ce qui concerne ce dernier, il s'agit d'un implant constitué d'une base en couches permettant sa fixation dans une découpe en forme de fenêtre pratiquée dans le cartilage thyroïdien et d'un élément en saillie, en forme de triangle ou « d'aileron de requin », qui vient appuyer sur le muscle de la corde vocale. Cet implant est décrit dans la demande de brevet publiée sous la référence WO 92/21303. Comme il ressort de la description de cette demande de brevet, une des caractéristiques de l'implant dit « de Montgomery » réside dans la forme même de la base de fixation qui comprend plusieurs couches, une couche inférieure, une couche intermédiaire et une couche supérieure, la couche inférieure et la couche supérieure ayant une surface plus grande que la couche intermédiaire afin de faciliter l'ancrage de la base dans la fenêtre pratiquée au sein du cartilage thyroïdien. Un exemple de cet implant est représenté à la figure 1 et sera discuté plus loin.

Il est donc nécessaire de pratiquer une ouverture de grande taille au sein du cartilage thyroïdien afin de permettre le passage d'un tel implant. En outre, le patient subit un traumatisme important au niveau de ce cartilage du fait que ce dernier ne peut se refermer complètement, la couche intermédiaire le traversant de part en part. Par ailleurs, de par un tel ancrage, il existe des risques d'inconfort, pouvant aller jusqu'à l'arrachement du cartilage, en cas de déplacement ou de pression trop élevée.

Enfin, cet implant étant réalisé en silicone et ayant une taille unique, il est nécessaire d'utiliser un instrument de mesure qui comporte un élément en saillie de forme similaire à celui de l'implant et un élément d'arrêt pour déterminer la taille d'implant optimale à mettre en place. Une fois la taille déterminée, il convient alors de retirer l'appareil de mesure et de mettre en place l'implant présentant les dimensions les plus proches des mesures réalisées.

Non seulement, le recours nécessaire à une étape de mesure, augmente les manipulations effectuées par le praticien ainsi que, par conséquent, les risques et désagréments liés à l'opération, mais le choix d'un implant parmi un éventail limité ne permet pas d'assurer la sélection d'un implant correspondant exactement à l'espace à combler entre le cartilage thyroïdien et la corde vocale à réparer. Enfin, il est nécessaire de contrôler régulièrement pour vérifier que la taille de l'implant mis en place est toujours la plus adaptée.

L'implant décrit dans le document US5306298 es un implant universel comprenant un premier élement qui prend appui sur la corde vocale, un deuxième élément fixé sur la paroi externe du cartilage thyroïdien et de d'ajustement permettant de règler la pression sur la corde vocale.

La présente invention a pour objectif de pallier ces inconvénients en proposant un implant universel, réglable, capable de s'adapter à toutes les configurations et dimensions, plus particulièrement à la distance entre le cartilage thyroïdien et le muscle de la corde vocale.

Plus particulièrement, selon un premier aspect, l'invention a pour objet un implant universel venant se positionner entre le cartilage thyroïdien et une corde vocale de manière à déplacer cette dernière en vue de restaurer la parole chez un patient, ledit implant comprenant
i) un premier élément formant « prothèse » venant prendre appui sur le muscle de la corde vocale entraînant une force de réaction R du muscle de la corde vocale sur ladite prothèse,
ii) un deuxième élément formant « contreplaque » venant prendre appui sur la paroi interne du cartilage thyroïdien entraînant une force de réaction R' du cartilage thyroïdien sur ladite contreplaque, et
iii) au moins un troisième élément de liaison de la prothèse et de la contreplaque,
   ladite contreplaque étant mobile sur ledit au moins un élément de liaison, permettant ainsi l'ajustement de la distance entre la prothèse et la contreplaque de manière à pouvoir ajuster les forces de réaction R et R' exercées respectivement par le muscle de la corde vocale et par le cartilage thyroïdien pour maintenir l'implant en place.

L'implant objet de l'invention est révolutionnaire en ce sens qu'il permet pour la première fois, contrairement à l'ensemble des préjugés de l'homme de l'art, d'éviter qu'une portion de l'implant traverse le cartilage thyroïdien. En effet, l'implant objet de l'invention est maintenu en place uniquement du fait de l'action des forces de réaction R et R' exercées respectivement par le muscle de la corde vocale et le cartilage thyroïdien.

Plus particulièrement, ladite force de réaction R s'exerce substantiellement perpendiculairement au plan AA' défini par l'axe longitudinal de la corde vocale tandis que ladite force de réaction R' s'exerce substantiellement perpendiculairement au plan BB' défini par la surface de la paroi interne du cartilage thyroïdien.

Cette stabilité de l'implant selon l'invention lui est acquise de part sa capacité a être réglable.

Dans la pratique, le fait de pouvoir régler de manière très précise la distance D entre la prothèse et la contreplaque permet de moduler de manière très précise les forces de réaction R et R' de sorte qu'elles soient suffisantes pour maintenir l'implant en place sans toutefois entraîner de gène pour le patient ni de dégradation du cartilage thyroïdien ou du muscle de la corde vocale.

En outre, un autre avantage de l'implant selon l'invention est que ce dernier peut être ajusté au cours du temps de manière à s'adapter à toute nouvelle déformation du muscle de la corde vocale

Avec les implants utilisés à ce jour, il était nécessaire de pratiquer une opération lourde afin de retirer l'implant en place pour pouvoir le remplacer par un implant de plus grande taille en cas de nouvelle distension du muscle de la corde vocale ou de relâchement du cartilage thyroïdien ou bien par un implant de plus petite taille en cas de durcissement du muscle de la corde vocale ou du cartilage thyroïdien. Une telle opération, on le comprendra bien, représente une gêne et des risques supplémentaires pour le patient.

Ces inconvénients importants n'existent plus avec l'implant objet de l'invention. En effet, pour les besoins d'ajustement il n'est plus nécessaire de pratiquer une telle opération de remplacement de l'implant, celui-ci pouvant tout simplement être réglé par une action adéquate au niveau de l'élément de liaison afin d'augmenter la distance entre la prothèse et la contreplaque, appelée distance D, et ainsi régler correctement l'implant de manière à pallier à toute distension du muscle de la corde vocale ou du cartilage thyroïdien.

Il est bien évidemment possible, en cas de durcissement du muscle de la corde vocale par exemple, d'effectuer l'opération inverse en diminuant la distance D entre la prothèse et la contreplaque.

Il faut bien comprendre que l'invention repose sur le principe du déplacement indirect de la prothèse. De manière plus précise, la contreplaque qui est mobile sur l'élément de liaison vient se positionner en appui sur le cartilage thyroïdien. Lorsque le chirurgien agit sur l'élément de liaison de manière à augmenter la distance D, la contreplaque va se déplacer vers le cartilage thyroïdien. Ce dernier étant rigide, il va s'opposer au déplacement de la contreplaque, ce qui aura pour effet d'augmenter la force de réaction R' du cartilage sur ladite contreplaque avec pour conséquence un déplacement dans le sens inverse de l'ensemble de l'implant du fait de la résistance du cartilage thyroïdien. Il s'en suit une augmentation de la force de réaction R de la corde vocale sur l'implant objet de l'invention. Le résultat final étant que l'implant est solidement maintenu coincé entre le cartilage thyroïdien et le corde vocale.

En agissant de manière inverse sur l'élément de liaison, cela aboutit également, de manière indirecte, à une diminution de la force de réaction R exercée par la corde vocale.

Selon une forme d'exécution donnée de l'invention, l'implant selon l'invention est caractérisé en ce que chacun des trois éléments consiste en une pièce distincte.

Une telle forme d'exécution a pour avantage de permettre le cas échéant de ne remplacer qu'une partie de l'implant. En outre, cela facilite la mise en place de l'implant lors de l'opération chirurgicale.

Toutefois, il est bien entendu que toute réalisation en une ou deux pièces distinctes doit également être comprise comme faisant partie intégrante de l'invention.

Sera décrit par la suite un mode de réalisation préféré de l'invention.

Plus particulièrement, l'implant selon l'invention est caractérisé en ce que ledit au moins un élément de liaison consiste en une tige comprenant une portion filetée, ladite portion filetée venant coopérer avec la contreplaque formant écrou de manière à pouvoir déplacer cette dernière et en ajuster la distance D d'avec la prothèse.

Le fait de n'utiliser qu'un élément de liaison apparaîtra évident à l'homme de l'art, en ce sens qu'il permet de limiter les éléments étrangers au corps humain. Cela permet également de diminuer le coût de production de l'implant. Toutefois, le recours à plusieurs éléments de liaison, bien que peu conseillé, reste couvert par la présente demande de brevet.

Dans cette forme d'exécution, il faut comprendre que la tige formant élément de liaison présente au moins une portion filetée faisant office de vis de manière à coopérer avec un orifice fileté prévu à cet effet au sein de la contreplaque. Il en résulte que ladite contreplaque est mobile en translation le long de l'élément de liaison lorsque celui-ci est en rotation, à la manière d'un écrou le long d'une vis.

Selon une forme de réalisation encore plus préférée, il est prévu au niveau de la section de l'extrémité filetée au moins un élément de coopération avec un outil destiné à pouvoir agir sur l'élément de liaison. Plus particulièrement, cet élément de coopération consiste en une empreinte mâle ou femelle notamment une encoche permettant l'accouplement à un outil, comme par exemple un tournevis, permettant d'imprimer un mouvement de rotation à la tige formant élément de liaison. Par une action en rotation sur l'élément de liaison, il devient alors possible de déplacer la contreplaque en translation. Dans là pratique, et contrairement aux implants de l'art antérieur, il sera suffisant de pratiquer une toute petite ouverture au sein du cartilage thyroïdien pour pouvoir passer un tournevis et ainsi régler la distance D.

Bien évidemment, tout dispositif équivalent permettant un réglage de la longueur de l'élément de liaison reste couvert par la présente invention.

Selon une autre forme de réalisation, il pourrait être envisagé, par exemple, d'utiliser un dispositif comprenant des systèmes à mémoire de forme comme des ressorts pouvant être comprimés lors de l'installation de l'implant, ces derniers se relâchant une fois l'implant en place de manière à générer des forces de réaction R et R' pour assurer le maintient de l'implant en place. Des systèmes comprenant des aimants ou une crémaillère pourraient également être envisagés.

Une autre caractéristique de l'invention est que, lors du mouvement de rotation de la tige formant élément de liaison, il est nécessaire que la contreplaque ainsi que la prothèse ne soient ni l'une, ni l'autre entraînées par ce mouvement et se mettent alors à tourner. Cela aurait pour effet d'altérer le cartilage thyroïdien mais surtout le muscle de la corde vocale.

Pour ce qui est de la contreplaque, lors de la rotation de la tige formant élément de liaison, la force de réaction R' exercée par le cartilage thyroïdien augmente. Il en résulte une augmentation des forces de frottement, ce qui a pour effet d'entraver toute rotation de la contreplaque sur elle-même autour de la tige formant élément de liaison.

Pour ce qui est de la prothèse, il est prévu un moyen de coopération avec la tige formant élément de liaison permettant d'éviter tout entraînement de la prothèse en rotation.

Plus particulièrement, l'implant selon l'invention est caractérisé en ce que ladite tige filetée formant élément de liaison comprend, au niveau de l'extrémité opposée à l'extrémité filetée coopérant avec la contreplaque, une portion lisse venant s'insérer dans un logement prévu à cet effet au sein de la prothèse de manière à pouvoir tourner librement sans entraîner la rotation de la prothèse.

Le fait que la tige formant élément de liaison présente une surface lisse et vienne s'insérer librement au sein d'un logement également lisse sur sa surface interne permet, de ne pas rendre ces deux éléments solidaires ; ces derniers ne pouvant alors coopérer dans le cadre de la rotation de l'un ou de l'autre. Bien entendu il ne s'agit que d'une illustration d'un mode de réalisation, la non coopération en rotation de la tige formant élément de liaison et de la prothèse pouvant être obtenue de diverses autres manières notamment par l'utilisation d'un roulement.

D'autre parut, cette surface lisse de la même extrémité de l'élément de liaison peut s'étendre au-delà de la partie insérée dans le logement de la prothèse, créant une interruption du filetage et une augmentation de la section de l'élément de liaison qui empêche que l'élément de liaison puisse être sorti accidentellement de la contreplaque en effectuant une opération de dévissage.

Dans la pratique, selon une forme de réalisation préférée, les dimensions du logement au sein de la prothèse sont légèrement plus grandes que celles de la section de la tige formant élément de liaison afin d'éviter toute force de frottement entre ces deux éléments. Il s'en suit que la tige formant élément de liaison tournera librement au sein dudit logement sans entraîner avec elle la prothèse.

L'élément de liaison, s'il n'entraîne pas de rotation de la prothèse suite à sa propre rotation sera cependant déplacé par translation et viendra alors pousser ladite prothèse entraînant également un mouvement de translation de cette dernière.

Selon une forme de réalisation supplémentaire, il peut être envisagé de pouvoir facilement diminuer la distance D.

Pour ce faire, ledit logement peut comprendre un dispositif de maintien de l'élément de liaison une fois ce dernier inséré.

Tout dispositif permettant de solidariser la tige formant élément de liaison avec la prothèse de manière à ce que, lors du retrait de la tige formant élément de liaison cette dernière ne sorte pas du logement mais entraîne la prothèse dans sa translation doit être considéré comme faisant partie intégrante de l'invention.

Un mode de réalisation préféré consiste en ce que ledit dispositif de maintien soit un épaulement prévu au sein du logement, ledit épaulement coopérant avec au moins un élément faisant saillie au niveau de l'extrémité lisse de l'élément de liaison.

Un autre mode de réalisation peut consister à prévoir un système de crochets ou bien tout simplement à utiliser des pièces aimantées.

Selon un autre aspect de l'invention, l'implant selon l'invention est caractérisé en ce que ladite prothèse est réalisée en titane poreux, préférentiellement microporeux.

L'utilisation de titane microporeux a pour avantage de permettre une colonisation de la prothèse par les cellules environnantes tout en limitant les rejets de l'implant par l'organisme. Il constitue un support rigide de croissance et est donc appelé à rester plus ou moins définitivement dans l'organisme. Ce point est d'une grande importance puisqu'il permet de pallier un inconvénient majeur des prothèses en silicone utilisées à ce jour. En effet, le silicone se dégradant rapidement et ne permettant pas une colonisation adéquate de l'implant, il est nécessaire d'en contrôler l'état régulièrement et de changer l'implant le cas échéant, avec les désagréments et les risques que cela implique pour le patient.

De manière générale, un tel matériau est constitué d'une juxtaposition tridimensionnelle de billes de titane non allié (préférentiellement comme le titane T40 ou tout alliage de titane biocompatible) dont le diamètre est généralement compris entre 200 et 650 micromètres. Les cavités entre les billes sont interconnectées réalisant alors une porosité ouverte généralement de l'ordre de 30 à 40 %. Peut être citée, comme illustration d'un tel matériau, la demande de brevet français n° 2 758 974 ou le brevet européen EP 0 856 299 B1. Bien évidemment, les éléments ci-dessus sont donnés à titre d'exemple illustratif et tout matériau équivalent connu de l'homme de l'art peut être utilisé selon l'invention notamment tout matériau biocompatible tels que les mousses de titane, le titane massif, le corail, le silicone, la poudre d'os.

La réalisation de la prothèse en titane microporeux peut se décliner en de nombreux modes de réalisation. Il peut notamment être envisagé, sans que cela soit obligatoire, de noyer dans la base de la prothèse une plaque en titane massif percée d'un trou lisse formant le logement dans lequel la portion lisse de la tige filetée vient se loger. Dans un mode de réalisation plus économique le logement est pratiquée directement dans la prothèse en titane poreux sans adjonction d'une quelconque autre pièce.

La présente invention vise également, de manière générale, l'utilisation d'un implant selon l'invention pour le traitement de dysfonctionnements des cordes vocales.

Plus particulièrement, de tels dysfonctionnements consistent en une paralysie totale ou partielle des cordes vocales.

Enfin, selon un dernier aspect, la présente invention revendique également un procédé simple et peu contraignant de mise en place d'un tel implant.

Plus particulièrement, la présente invention concerne enfin un procédé de mise en place et/ou de réglage d'un implant selon l'invention. Ledit procédé consistant à positionner ledit implant de manière à ce que la contreplaque (5) vienne former un point d'appui contre la paroi interne du cartilage thyroïdien et
- i): entraîne le déplacement de l'élément de liaison (7) de manière à augmenter la distance D augmentant ainsi, compte tenu de la résistance offerte par ledit cartilage thyroïdien, la force de réaction R', ce qui a pour effet de déplacer ladite prothèse entraînant ainsi une augmentation de la pression exercée sur le muscle de la corde vocale ; ou
- ii): entraîne le déplacement dudit élément de liaison de manière à diminuer la distance D diminuant ainsi la force de réaction R', ce qui a pour effet, compte tenu de la force de réaction R exercée par le muscle de la corde vocale sur l'ensemble formé par ledit au moins un élément de liaison et par ladite prothèse, de diminuer la pression exercée sur le muscle de la corde vocale.

L'invention sera mieux comprise à la lecture des exemples de réalisation décrits ci-dessous en combinaison avec les figures suivantes dans lesquelles :
La figure 1 illustre l'art antérieur et représente un implant de Montgomery en coupe transversale,
La figure 2 représente une forme d'exécution préférée d'un implant en coupe transversale selon l'invention,
La figure 3 représente l'implant selon l'invention tel que mis en place sur un patient, et
La figure 4 représente, plus en détails, la disposition de l'implant selon l'invention entre le cartilage thyroïdien et la corde vocale endommagée.

Comme décrit plus haut, et comme il ressort de la figure 1, l'implant décrit dans le document de l'art antérieur le plus proche, à savoir l'implant de Montgomery, comprend une portion sensiblement en forme d'aileron de requin destinée à venir exercer une pression sur la corde vocale endommagée et une seconde portion destinée à maintenir l'ensemble de l'implant en place au sein du cartilage thyroïdien. Cette seconde portion comprend elle-même trois parties, une partie centrale insérée au travers même du cartilage thyroïdien et deux autres parties, respectivement de chaque côté de ladite partie centrale, venant prendre appui sur chacune des faces du cartilage thyroïdien de manière à faire office de butée et ainsi maintenir l'implant en place.

Comme il ressort de la figure 2, l'implant 1 selon l'invention présente une forme tout à fait innovante en ce sens qu'il comprend un premier élément formant prothèse 3, un deuxième élément formant contreplaque 5 et un troisième élément formant élément de liaison 7. L'élément de liaison 7 comprend une portion filetée, à la manière d'une vis, qui vient se visser au sein d'un orifice 11 prévu au sein de la contreplaque 5, ledit orifice 11 étant également fileté sur ses parois internes, à la manière d'un écrou. Au niveau de l'extrémité de la portion filetée de l'élément de liaison 7, est également prévue une encoche 13. Il est alors possible de déplacer l'élément 7 au travers de la contreplaque 5 à la manière d'une vis dans un écrou par simple action à l'aide d'un tournevis venant s'insérer dans l'encoche 13 prévue à cet effet.

L'extrémité opposée de l'élément de liaison 7 vient s'encastrer au sein d'un logement 9 prévu à cet effet au sein de la prothèse 3. Comme il ressort de la figure 2, l'élément de liaison 7 tourne librement au sein du logement 9. Pour ce faire, les dimensions du logement 9 sont légèrement supérieures à celles de l'extrémité de l'élément de liaison 7 de manière à ce que ce dernier puisse tourner avec le minimum de frottement sur les parois internes dudit logement 9.

Le principe de l'implant 1 selon l'invention apparaît donc clairement à la vue de cette figure 2, à savoir qu'en agissant sur l'élément de liaison 7, cela devrait avoir pour effet d'entraîner la rotation de la contreplaque 5 augmentant ainsi la distance D. La contreplaque 5 étant maintenue en place contre le cartilage thyroïdien (non représenté), elle ne pourra pas se déplacer ni entrer en rotation , ce qui aura pour effet de déplacer l'élément de liaison 7 dans le sens inverse qui viendra alors pousser la prothèse 3 entraînant alors une augmentation de la pression exercée par la prothèse 3 sur le muscle de la corde vocale endommagée (non représentée). Autrement exprimé en visant, ou dévissant selon le sens du filetage, l'élément de liaison (7) sur la contreplaque 5 l'élément de liaison 7 déplace la prothèse 3 vers le muscle de la corde vocale qui se trouve elle-même déplacée et rapprochée de la seconde corde vocale. L'utilisation d'une vis permet ainsi de régler avec précision la position du muscle de la corde vocale endommagée afin de lui permettre de retrouver sa fonctionnalité.

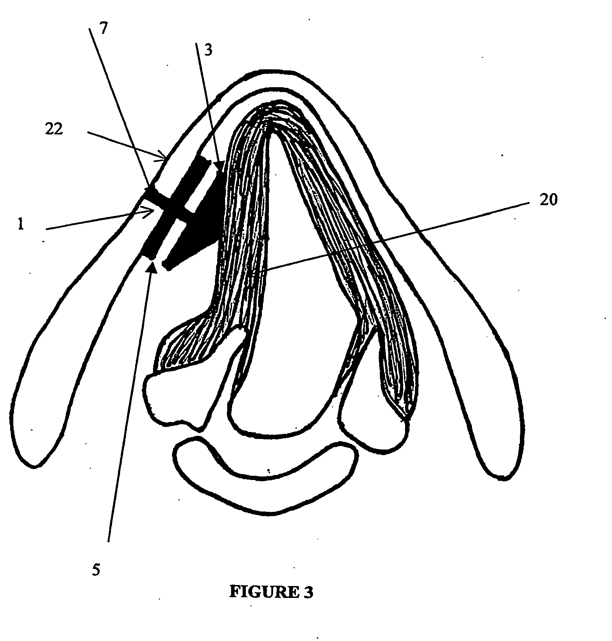

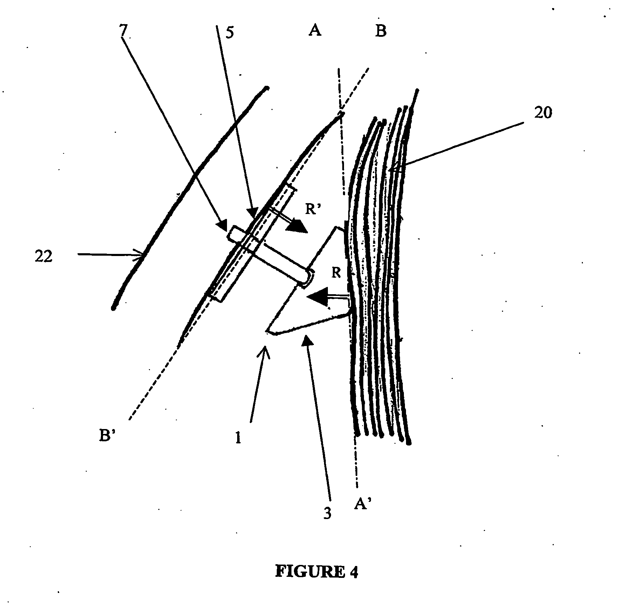

Le fonctionnement de la prothèse objet de la présente invention ressort plus clairement des figures 3 et 4 décrites ci-après.

Selon la forme d'exécution représentée, la prothèse 3 présente, en coupe transversale, un profil général en forme de triangle isocèle. Plus particulièrement, les dimensions envisagées pour la base d'un tel triangle peuvent être comprises entre 10 et 15 mm, et tout préférentiellement dans l'exemple représenté de 13 mm. La hauteur, ou médiane, dudit triangle peut être comprise entre 4 et 6 mm. De manière préférée, au moins le sommet dudit triangle venant appuyer sur le muscle de la corde vocale est arrondi afin d'éviter toute détérioration de ladite corde vocale. Il peut être envisagé un rayon de courbure pour ce sommet de l'ordre de 3 mm. Bien entendu, il faut comprendre que l'ensemble des dimensions données ci-dessus ne représente qu'un exemple illustratif d'un mode de réalisation et que ces dimensions ne sont aucunement à prendre comme limitatives.

La figure 3 est une vue générale, en coupe horizontale, de l'implant 1 selon l'invention monté entre le cartilage thyroïdien 22 et le muscle de la corde vocale endommagée 20 de manière à déplacer ce dernier.

Il ressort clairement de cette figure que seule une petite portion de l'élément de liaison 7 peut rester insérée au sein du cartilage thyroïdien 22. Il suffira alors au chirurgien de pratiquer une légère entaille au sein de ce cartilage 22 pour pouvoir glisser un tournevis et ainsi « retendre », ou « détendre », l'implant 1 afin d'ajuster la distance D entre la prothèse 3 et la contreplaque 5. Cet aspect présente un avantage considérable par rapport à l'art antérieur où il était nécessaire de pratiquer une grande ouverture au sein du cartilage thyroïdien 22 afin de pouvoir retirer l'implant en place dans son intégralité pour le remplacer par un nouvel implant de la taille approximativement appropriée.

La figure 4 représente un grossissement de la figure 3. Plus particulièrement, cette figure montre l'implant 1 positionné chez un patient.

La contreplaque 5 est en appui le long du cartilage thyroïdien 22 selon sensiblement le plan BB'. La force de réaction exercée par le cartilage thyroïdien 22 sur la contreplaque 5 est représentée par le vecteur R'. À l'opposé de la contreplaque 5, la prothèse 3 vient prendre appui, quant à elle, sur le muscle de la corde vocale à retendre 20 selon sensiblement le plan AA'. Cette dernière exerce alors une force de réaction sur la prothèse 3, ladite force étant représentée par le vecteur R. Il faut noter que la représentation des forces de réaction R et R' sous la forme de vecteurs n'est qu'illustrative afin de comprendre le principe de l'invention ; les dimensions des ces vecteurs R et R' sur la présente figure 4 ne sont donc pas à prendre en considération. Il est alors évident pour l'homme de l'art qu'en ajustant la distance D entre la contreplaque 5 et la prothèse 3, il est possible de modifier et moduler les forces de réactions R et R' de manière non seulement à maintenir l'implant 1 en place mais également à déplacer suffisamment la corde vocale 20 par pression de manière à restaurer, au moins en partie, la parole au patient.

Les modes de réalisation de l'invention décrits ci-dessus consistent, bien entendu, en des modes de réalisation préférés. Tout perfectionnement n'entraînant pas de modification substantielle du principe de l'invention doit être considéré comme couvert par la présente demande de brevet et, tout particulièrement, par les revendications ci-après qui en définissent la portée.

## Revendications

1. (modifiée) Implant universel (1) venant se positionner entre le cartilage thyroïdien (22) et une corde vocale (20) de manière à déplacer cette dernière en vue de restaurer la parole chez un patient, comprenant :
i) un premier élément formant « prothèse » (3) venant prendre appui sur le muscle de la corde vocale (20) entraînant une force de réaction R de la corde vocale sur ladite prothèse,
ii) un deuxième élément formant « contreplaque » (5) venant prendre appui sur la paroi interne du cartilage thyroïdien (22) entraînant une force de réaction R' du cartilage thyroïdien sur ladite contreplaque, et
iii) au moins un troisième élément de liaison (7) de la prothèse (3) et de la contreplaque (5),
ladite contreplaque (5) étant mobile sur ledit au moins un élément de liaison (7), permettant ainsi l'ajustement de la distance D entre la prothèse (3) et la contreplaque (5) de manière à pouvoir ajuster les forces de réaction R et R' exercées respectivement par le muscle de la corde vocale (20) et par le cartilage thyroïdien (22) pour maintenir l'implant (1) en place.

2. Implant (1) selon la revendication 1, **caractérisé en ce que** chacun des trois éléments (3, 5, 7) consistent en des pièces distinctes.

3. Implant (1) selon l'une quelconque des revendications précédentes, **caractérisé en ce que** ledit au moins un élément de liaison (7) consiste en une tige comprenant une portion filetée, ladite portion fileté venant coopérer avec la contreplaque (5) formant écrou de manière à pouvoir déplacer l'élément de liaison (7) et ajuster la distance D comprise entre la contreplaque (5) et la prothèse (3).

4. Implant (1) selon l'une quelconque des revendications précédentes, **caractérisé en ce que** ladite tige filetée formant élément de liaison (7) comprend, au niveau de l'extrémité opposée à l'extrémité filetée coopérant avec la contreplaque (5), une portion lisse venant se loger dans un logement (9) prévu à cet effet au sein de la prothèse (3) de manière à pouvoir tourner librement sans entraîner la rotation de la prothèse (3).

5. Implant (1) selon l'une quelconque des revendications précédentes, **caractérisé en ce que** ladite prothèse (3) est réalisée en titane poreux, préférentiellement microporeux.

6. Implant (1) selon la revendication 5, **caractérisé en ce que** ladite prothèse (3) comporte un élément massif percé d'un trou formant le logement (9).

## Claims

1. A universal implant (1) positioned between the thyroid cartilage (22) and a vocal cord (20), displacing the latter in order to restore the patient's speech, including:
i) a first element forming a "prosthesis" (3) resting on the muscle of the vocal cord (20) generating a reaction force R of the vocal cord on said prosthesis,
ii) a second element forming a "counterplate" (5) resting on the inner wall of the thyroid cartilage (22) generating a reaction force R' of the thyroid cartilage on said counterplate, and
iii) at least a third linking element (7) of the prosthesis (3) and the counterplate (5),
said counterplate (5) being mobile on said at least one linking element (7), thereby enabling the adjustment of the distance D between the prosthesis (3) and the counterplate (5) in order to adjust the reaction forces R and R' exerted respectively by the muscle of the vocal cord (20) and by the thyroid cartilage (22), in order to hold the implant (1) in place.

2. Implant (1) according to claim 1, wherein each of the three elements (3, 5, 7) consists of distinct pieces.

3. Implant (1) according to any of the preceding claims, wherein said at least one linking element (7) consists of a rod comprising a threaded portion, said threaded portion interacts with the counterplate (5) forming a nut, making it possible to displace the linking element (7) and adjust the distance D between the counterplate (5) and the prosthesis (3).

4. Implant (1) according to any of the preceding claims, wherein said threaded rod forming the linking element (7) comprises, on the tip opposite the threaded tip interacting with the counterplate (5), a smooth portion fitting into a housing (9) provided within the prosthesis (3) in order to rotate freely without causing the rotation of the prosthesis (3).

5. Implant (1) according to any of the preceding claims, wherein said prosthesis (3) is made of porous titanium, preferably microporous.

6. Implant (1) according to claim 5, wherein said prosthesis (3) comprises a solid element provided with a hole forming the housing (9).

## Patentansprüche

1. Universalimplantat (1), das zwischen Schildknorpel (22) und einem der Stimmbänder (20) eingesetzt wird, wodurch letzteres verschoben und die Stimmgebungsfähigkeit des Patienten wiederhergestellt wird. Das Implantat besteht aus:
i) einem als "Prothese" (3) dienenden ersten Teil, der am Stimmbandmuskel (20) anliegt und beim Stimmband eine Reaktionskraft R auf diese Prothese hervorruft,
ii) einem als "Gegenstück" (5) dienenden zweiten Teil, der an der Innenwand des Schildknorpels (22) anliegt und beim Schildknorpel eine Reaktionskraft R' auf dieses Gegenstück hervorruft,
iii) zumindest einem weiteren Verbindungsteil (7) zwischen Prothese (3) und Gegenstück (5),
wobei das Gegenstück (5) beweglich an diesem einen oder mehreren Verbindungsteile(n) (7) aufliegt. Dadurch besteht die Möglichkeit, den Abstand D zwischen Prothese (3) und Gegenstück (5) dergestalt einzustellen, dass die Reaktionskräfte R und R', welche vom Stimmbandmuskel (20) und vom Schildknorpel (22) ausgehen, das Implantat (1) in Stellung halten.

2. Implantat (1) gemäß Anspruch 1, **dadurch gekennzeichnet, dass** die drei Teile (3, 5, 7) drei Einzelteile sind.

3. Implantat (1) nach einem beliebigen vorstehenden Anspruch, **dadurch gekennzeichnet, dass** der Verbindungsteil oder die Verbindungsteile (7) aus einem Gewindestab bestehen, dessen Gewinde mit dem Gegenstück (5) wie mit einer Mutter zusammenspielt, wodurch der Verbindungsteil (7) verschoben und der Abstand D zwischen Gegenstück (5) und Prothese (3) angepasst werden kann.

4. Implantat (1) nach einem beliebigen vorstehenden Anspruch, **dadurch gekennzeichnet, dass** der als Verbindungsteil dienende Gewindestab (7) an seinem anderen Ende, welches dem im Zusammenspiel mit dem Gegenstück (5) befindlichen Gewinde gegenüberliegt, einen glatten Abschnitt besitzt, welcher in einer entsprechenden, in der Prothese (3) dafür vorgesehenen Aufnahmeöffnung (9) ruht, in welcher er sich drehen kann ohne die Prothese (3) mitzudrehen.

5. Implantat (1) nach einem beliebigen vorstehenden Anspruch, **dadurch gekennzeichnet, dass** selbige Prothese (3) aus porösem und möglichst mikroporösem Titan besteht.

6. Implantat (1) nach Anspruch 5, **dadurch gekennzeichnet, dass** selbige Prothese (3) über einen massiven Teil verfügt, in dem eine Bohrung als Aufnahmeöffnung (9) vorgesehen ist.
